# EUROPEAN PATENT APPLICATION

(11) **EP 1 254 954 A1**
(43) Date of publication of application: **06.11.2002**
(21) Application number: 01902796.0
(22) Date of filing: 07.02.2001
(51) Int. Cl.: C12N 15/09, C12Q 1/68, C12Q 1/02, G01N 33/566, G01N 33/50, G01N 33/15

(54) **METHOD OF DETECTING LIGAND OR LIGAND-LIKE LOW-MOLECULAR WEIGHT COMPOUND**

(30) Priority: 08.02.2000 JP 2000030039; 06.10.2000 JP 2000307653
(71) Applicant: SSP Co., Ltd., Chuo-ku, Tokyo 103-8481 (JP)
(72) Inventor: TANIYAMA, Tadayoshi, Yokohama-shi, Kanagawa 231-0864 (JP); NISHIMURA, Tadahiro, Narashino-shi, Chiba 275-0026 (JP); KUSANO, Koji, Yachiyo-shi, Chiba 276-0045 (JP); EBARA, Shinji, Narita-shi, Chiba 286-0042 (JP); TACHIBANA, Koichi, Narita-shi, Chiba 286-0041 (JP)
(74) Representative: Hartz, Nikolai, Dr.
(86) International application number: JP0100858
(87) International publication number: WO01059096

(57) **Abstract**

A method of detecting a ligand or a ligand-like low-molecular weight compound from among test compounds characterized by separating a ligand-dependent cell line having a ligand receptor gene and an antibiotic resistance gene transferred thereinto from other cell lines, culturing the cell line in the presence of the test compounds and then examining the proliferation ability of the cells. Use of this method makes it possible to exactly screen a ligand (a physiologically active substance) or a low-molecular weight compound having the same action by a simple procedure.

## Description

### Technical Field

This invention relates to a method for assaying a ligand or a ligand-like low molecular compound by using as an index cytoproliferative activity of a ligand-dependent cell line transfected with a ligand receptor gene.

### Background Art

A variety of ligands (physiologically active substances), which take part in the immune system, the hematopoietic system, the endocrine system and the nervous system, are known to bind to specific receptors existing on cell membranes of respective competent cells. It has been considered that stimuli from the outside of the cell membranes are transmitted as information into the cells via the binding and induce proliferation or differentiation of the cells to maintain high-order functions *in vivo.*

As many as 30 types of cytokines, growth factors and the like have been identified as ligands to date. For example, granulocyte colony stimulating factor (G-CSF) is a growth factor produced from monocytes and macrophages, fibroblasts, endotheliocytes or the like. This factor is known to act on neutrophilic precursors and induces their proliferation, differentiation and exaltation and as a result, to guard the organism from pathogens and to have antitumor activities [Asano et al., "JIKKEN IGAKU (Experimental Medicines)", **7**(15), 1859-1865 (1989)]. Accordingly, ligands are expected to be applicable not only to neutropenia, intractable infectious diseases and bone marrow hypofunction but also to AIDS.

Interleukin 10 (IL-10), on the other hand, is known to be a factor which is produced from Th2 cells and inhibits cytokine from Th1 cells [Ishida et al. "RINSHO MENEKI (Clinical Immunology)", **27**(Suppl.16), 97-106 (1995)]. In recent years, this IL-10 has been found to be produced from various cells such as activated B cells, macrophages, keratinocytes, mast cells and the like. This interleukin is also known to inhibit expression of MHC class II antigen, B7(CD80,CD86), of monocytes or macrophages and also to inhibit expression of intercellular adhesion molecules (ICAM-1)[de Waal Malefyt et al., "J. Exp. Med.", **177**, 915 (1991)]. Further, it also has proliferation inhibiting effect against proliferation of T cells and activating effect for B cells [Ishida et al., "RINSHO MENEKI (Clinical Immunology)", **27**(Suppl.16), 97-106 (1995)]. In view of these effects, IL-10 is expected to find clinical applications to autoimmune diseases (rheumatoid arthritis, etc.), preparation for organ transplantation, post-transplant organ failure by inflammatory cytokines, inflammatory diseases and the like.

Further, erythropoietin (EPO) is produced primarily from paranephric tubular endotheliocytes, has such effect as acting on early erythroblast precursors and late erythroblast precursors to stimulate erythropoiesis by way of promotion of differentiation or proliferation into erythroblasts and mRNA elicitation of heme synthesis [Hirashima et al., "JIKKEN IGAKU (Experimental Medicines)", **7**(15), 1852-1858 (1989)], and are clinically applied to renal anemia and refractory anemia (aplastic anemia, refractory anemia) these days. Thrombopoietin (TPO), on the other hand, is produced mainly from sinusoid endotheliocytes of the liver, has such effect as acting on megakaryoblastic precursors and megakaryoblasts to stimulate induce the production of platelets from megakaryocytes [Teramura et al., "GAN TO KAGAKU RYOHO (Japanese Journal of Cancer and Chemotherapy), **26**(4), 421-428 (1999)], and are now expected to find clinical applications to thrombocytopenia, acute myelogenous leukemia, aplastic anemia, myelodysplastic syndrome and the like associated with carcinostatic chemotherapy, cancer radiation therapy or bone marrow transplantation.

Insulin is a physiologically active substance, which is produced from pancreatic β cells and is deeply concerned with diabetes. As a result of binding to insulin receptors on cell membranes, it enhances transport across the cell membranes, promotes uptake of sugar or amino acids into the cells, suppresses adipolysis in fatty tissues, and promotes synthesis of glycogen and proteins in muscles and the liver [Zawalich, W.S., et al., "Endocrinology", **103**, 2027-2034 (1978)]. As many as 280,000 diabetics are currently under insulinization therapy in Japan, and the number of such patients is steadily increasing every year.

Nerve growth factor (NGF) is synthesized in the cerebra cortex and hippocampus, acts on cells of peripheral nerves such as sympathetic nerves and sensory nerves, and is effective in function maintenance and survival maintenance [Mima et al., "IYAKU JOURNAL (Medicine and Drug Journal)", **26**(2), 245-249 (1990)]. Nerve growth factor is now expected to find clinical applications to Alzheimer's dementia, cerebral ischemic diseases, peripheral nerve damages and the like.

With respect to such ligands (physiologically active substances) as described above, their genes have recently been isolated one after one, thereby permitting provision of physiologically active substances of the gene recombinant type and resulting in identification of many peptides capable of acting as ligands (physiologically active substances).

These peptides, however, have not been used as drug except for an extremely small fraction of them, and only G-CSF, interferon α and the like are clinically applied. As reasons for the difficulty in using such peptides as drug, it has been indicated *inter* alia that, as they are proteins, they are susceptible to metabolism and are short in residual period and also that they tend to induce production of antibodies and hence to be neutralized.

With a view to overcoming these problems, screening is under way for low molecular compounds (hereinafter called "ligand-like low molecular compounds") capable of inducing similar action as these ligands (physiologically active substance) in place of the ligands which are proteins. However, the screening methods known to date are accompanied by a problem in that, as they make use of competent cells for the ligands (physiologically active substances), intricate tracing of signals must be performed subsequent to the binding of the ligands to their corresponding receptors although the assay of such signals is difficult. For example, quantitation of a substance produced from competent cells or measurement of signals or the like in the competent cells is needed, leading to a problem in that an extremely complex quantitation or measurement is required.

An object of the present invention is, therefore, to provide a method which permits accurate screening of a ligand (physiologically active substance) or a low molecular compound capable of acting in a similar way as the ligand by simple procedures.

### Disclosure of the Invention

The present inventors have proceeded with a variety of investigations on assay methods for ligands (physiologically active substances). As a result, it has been found that use of cells proliferable only in the presence of a ligand in place of competent cells for the ligand, said competent cells having been used in the conventional methods, makes it possible to easily determine the existence of the ligand or a ligand-like substance by checking proliferation of the cells, leading to the completion of the present invention.

The present invention, therefore, provides a method for assaying a ligand or a ligand-like low molecular compound in a test compound, which comprises separating a ligand-dependent cell line, which has been transfected with a ligand receptor gene and the antibiotic-resistant gene, from other cell lines, culturing the ligand-dependent cell line in the presence of the test compound, and then determining cytoproliferative activity of the ligand-dependent cell line.

The present invention also provides a method for assaying a ligand or a ligand-like low molecular compound in a test compound as described above, wherein the culture of the ligand-dependent cell line is conducted in the presence of the test compound and at least one of a reducing agent and interleukin 3 (IL-3).

The present invention further provides a reagent and a kit, which are useful in both of the above-described methods.

### Brief Description of the Drawings

FIG. 1 is a graph showing the results of an investigation in Example 1 on a relationship between the hG-CSF concentration in a culture medium and the degree of proliferation of a cell line transfected with an hG-CSF receptor gene.
FIG. 2 is a graph showing the results of an investigation in Example 2 on a relationship between the hIL-10 concentration in a culture medium and the degree of proliferation of a cell line transfected with an hIL-10 receptor gene.
FIG. 3 is a graph showing effects of a reducing agent on proliferation of a G-CSF-dependent cell line.
FIG. 4 is a graph showing effects of IL-3 on the proliferation of the G-CSF-dependent cell line.
FIG. 5 is a graph showing effects of a reducing agent and IL-3 on proliferation of a G-CSF-dependent cell line in which G-CSF was a G-CSF-like low molecular substance.
FIG. 6 is a graph showing effects of a reducing agent on proliferation of an IL-10-dependent cell line.
FIG. 7 is a graph showing effects of IL-3 on the proliferation of the IL-10-dependent cell line.
FIG. 8 is a graph showing the results of an investigation in Example 8 on a relationship between the hEPO concentration in a culture medium and the degree of proliferation of a cell line transfected with an hEPO receptor gene.
FIG. 9 is a graph showing effects of a reducing agent on proliferation of an EPO-dependent cell line.
FIG. 10 is a graph showing effects of IL-3 on the proliferation of the EPO-dependent cell line.

### Embodiments of the Invention

The term "ligand-dependent cell line" as used herein means a cell line which can grow and proliferate only when a ligand or a ligand-like low molecular compound, which may hereinafter be referred to as "ligand or the like", exists in a culture medium. Further, the term "ligand-like low molecular compound" means a low molecular compound, which has one or more of a property of specifically binding to receptors expressed on cell surfaces, a property of activating the receptors and a property of substituting for signals in cells and which acts on the organism as if it is a ligand.

Each ligand-dependent cell line for use in the present invention can be prepared by introducing a ligand receptor gene, which corresponds to a ligand or the like to be assayed, and an antibiotic-resistant gene into a cultured cell line.

Described specifically, the ligand-dependent cell line can be prepared by suspending the cultured cell line in a suitable buffer, adding a solution of a DNA, which has been transfected with the ligand receptor gene and the antibiotic-resistant gene, in an appropriate solvent to the suspension, and then introducing the DNA into the cultured cell line.

Illustrative of cultured cell lines usable in the present invention are interleukin 3 (IL-3)-dependent mouse cell lines, for example, BAF/B03 [Palacios, R., et al., "Cell",**41**,727-734(1985); Shi, Y., et al., "J. Immunol.", **159**, 5318-5328 (1997)]. Examples of buffers useful for suspending such cells can include K-PBS (30.8 mM NaCl, 120.7 mM KCl, 8.1 mM Na₂HPO₄, 1.46 mM KH₂PO₄, 5mM MgCl₂).

As the ligand receptor gene to be inserted into such cells, it is possible to use, for example, those obtained by the methods described in the following papers:
- Human granulocyte colony stimulating factor receptor:
   Larsen, B.A., et al., "J. Exp. Med.", **172**, 1559-1570 (1990).
- Human interleukin 10 receptor:
   Liu, Y., et al., "J. Immunol.", **152**, 1821-1829 (1994).
- Human erythropoietin receptor:
   Jones, S. S., et al., "Blood", **76**, 31-35 (1990).
- Human thrombopoietin receptor:
   Mignotte, V., et al., "Genomics", **20**, 5-12 (1994).
- Human insulin receptor:
   Ebina, Y., et al., "Cell". **40**, 747-758 (1985).
- Human nerve growth factor receptor:
   Johnson, D., et al., "Cell", **47**, 545-554 (1986).

On the other hand, the antibiotic-resistant gene which has also been inserted in the cells is to facilitate the screening of cells with the ligand receptor gene introduced therein. Usable examples of the antibiotic-resistant gene can include puromycin-resistant genes, hygromycin-resistant genes, and neomycin-resistant genes. As these antibiotic-resistant genes, those obtained by processes described in the corresponding known papers can be used. However, plasmids with these antibiotic-resistant genes inserted therein, respectively, are already available. It is therefore possible to insert the above-described ligand receptor gene into these plasmids and to use the resultant plasmids as DNAs transfected with the ligand receptor gene and the antibiotic-resistant genes. Illustrative of such plasmids are pcDNA3.1, pCDNA3.1/Zeo, pcDNA3.1/Hygro, and pIRESpuro.

Upon lysing and inserting the DNA transfected with the ligand receptor gene and the antibiotic-resistant gene, use of a solvent commonly employed for lysing DNAs, for example, TE buffer (10 mM Tris-HCl, 1 mM EDTA; pH 8.0) is preferred.

As a process or method for introducing the ligand receptor gene and the antibiotic-resistant gene into the cultured cell line, a known process or method such as electroporation, the DEAE dextran process, the ribosome process or microinjection can be used. Of these, use of electroporation is preferred for its simplicity and high efficiency of gene introduction.

According to electroporation, high-voltage pulses are applied across a solution in which a cultured cell line and a DNA, which serves to encode the ligand receptor gene and the like, are suspended, whereby micropores are formed through the walls of cells of the cell line and the ligand receptor gene is introduced into the cells through the micropores. As specific conditions for electroporation, it is preferred to set the voltage at about 260 to 300 V and the capacitance at about 960 *µ* F. Concerning the introduction of a ligand receptor gene into the mouse cell line BAF/B03, a process has been reported by Rowlinson S.W., et al. Introduction of the ligand receptor can, therefore, be practiced with reference to the reported process ["J. Biological Chemistry", **273**(9), 5307-5314 (1998)].

The cell line with the ligand receptor gene introduced therein as described above is then cultured in the presence of the ligand and an antibiotic to separate it from cultured cells in which the ligand receptor gene has not been introduced. A culture medium usable here may be an ordinary culture medium. It is, however, essential for the culture medium to contain a ligand corresponding to the introduced ligand receptor and an antibiotic corresponding to the antibiotic-resistant gene, and no other ligand must be added.

In this culture, only the cell line transfected with the ligand receptor gene and the antibiotic-resistant gene is allowed to grow, and the cell lines not transfected with the ligand receptor gene die entirely. This makes it possible to separate the ligand-dependent cell line from the other cell lines. Unless the cell line were transfected with the antibiotic-resistant gene, even cell lines not transfected with the ligand receptor gene would have a chance to proliferate relying upon a component, for example, fetal calf serum (FCS) in a culture medium.

The ligand-dependent cell line constructed as described above is used for the assay of the ligand or ligand-like low molecular compound in the test compound. This assay method can be practiced by culturing the cell line in the presence of the test compound at an appropriate concentration and then determining the degree of proliferation of the cell line. As the ligand-dependent cell line is allowed to grow and proliferate only in the presence of the ligand or the ligand-like low molecular compound, proliferation of the ligand-dependent cell line makes it possible to determine the existence of the ligand or the ligand-like low molecular compound and as a consequence, to conclude that the compound is the ligand-like low molecular compound.

Upon conducting the above-described culture of the ligand-dependent cell line, it is preferred to have a reducing agent and optionally interleukin 3 (IL-3) included in addition to the test compound in the culture medium. Of these, the reducing agent acts to enhance the activity of the cells under culture and to promote their proliferation.

Although no particular limitation is imposed on the reducing agent, use of a reducing agent such as 2-mercaptoethanol or α-thioglycerol is preferred from the standpoint of action. The preferred amount of the reducing agent in the medium may range, but is not limited particularly to, from about 10⁻⁶ to about 10⁻³ M, notably from about 10⁻⁵ to about 10⁻⁴ M.

Further, IL-3 also acts to enhance the activity of the cells under culture and, upon practicing the method according to the present invention, can bring about an advantageous effect in that the proliferation of the cells under culture can be promoted.

As IL-3, IL-3 such as mouse recombinant IL-3 or a culture known to contain the same can be used. It can be added preferably at a concentration of from about 0.01 to about 100 ng/mL, notably from about 0.1 to about 10 ng/mL, in terms of IL-3.

Proliferation or non-proliferation of the ligand-dependent cells can be determined, for example, by culturing the ligand-dependent cell line for a predetermined time in a culture medium with the test compound contained therein and counting the cells after the culture. As preferred culture conditions, the culture may be effected, for example, at 37°C for about 48 hours in a 5%CO₂ incubator. Upon performing the counting of the cells after the culture, a reduction-type color-developing reagent is used as a colorimetric substance. Preferred examples of the reduction-type color-developing regents can include tetrazolium salts, with WST-1 [2-(4-iodophenyl)-3-(4-nitrophenyl)-5-(2,4-disulfo-phenyl)-*2H*-tetrazolium monosodium salt] being particularly preferred.

As electron carriers for such tetrazolium-based, color-developing reagents, phenazine methosulfate electron carriers are preferred, with combined use of 1-methoxy PMS (1-methoxy-5-methylphenazinium methylsulfate) being particularly preferred. A reagent kit for cell proliferation assay, which contains these WST-1 and 1-methoxy PMS in combination, is commercially available under the name of "Cell Counting Kit" from Dojindo Laboratories.

To practice the method of the present invention, it is preferred to use, for example, an assay reagent or kit for a ligand or a ligand-like low molecular compound, which contains the following components (a) to (d):
(a) a ligand-dependent cell line transfected with a ligand receptor gene and an antibiotic-resistant gene;
(b) a culture medium for said ligand-dependent cell line;
(c) a colorimetric substance; and
(d) an electron carrier.

Of these components, the component (a) and the component (b) are the cell line for use in the method of the present invention and a culture medium for allowing it to proliferate, respectively, while the component (c) and the component (d) are reagents for assaying proliferation of the cell line. A reducing agent and/or IL-3 may be incorporated in the component (b) out of the above components as needed.

When preparing the assay kit for the ligand or the like, these individual components can be provided separately or in the form of two or more reagents and eventually, can be combined together. Alternatively, as a reagent kit for cell proliferation assay in which WST-1, a colorimetric substance, and 1-methoxy PMS, an electron carrier, are combined is commercially available as described above, this reagent kit can be used instead of the component (c) and the component (d).

### Industrial Applicability

The conventional assays of a ligand or a ligand-like low molecular substance make use of competent cells for the ligand (physiologically active substance), and require procedures such as assay of signals or a product subsequent to binding of the ligand (physiologically active substance) to the receptor for the substance.

The method according to the present invention, on the other hand, makes use of a cell line obtained by inserting a ligand receptor gene into its cells and containing receptors for a physiologically active substance on the surfaces of the cells. The cells with the receptors expressed thereon cannot proliferate unless signals from the receptors are transmitted. In other words, these cells cannot survive unless the ligand (physiologically active substance) or its substitute compound binds to the receptors expressed on the surfaces of the cells.

As is appreciated from the foregoing, it has become possible to express the existence or non-existence of a ligand or a ligand-like low molecular compound in an assay system by the survival or death of the cells. This has now made it possible to quantitatively screen a great deal of the low-molecular compound based on a degree of proliferation of the cell line the determination of which is extremely easy compared with conventional determinations.

Accordingly, the present invention can promptly and easily assay a low molecular compound usable in place of a ligand (physiologically active substance) which takes part in the immune system, the hematopoietic system, the endocrine system or the nervous system, and is extremely useful for the development of drug capable of directly acting on the immune system, the hematopoietic system, the endocrine system or the nervous system, such as immunopotentiators.

### Examples

The present invention will be described in further detail based on the following examples. It should, however, be borne in mind that the present invention is by no means limited to or by these examples.

### Example 1

### Construction of a human G-CSF-dependent cell line

Using a primer prepared based on a human G-CSF receptor gene, a human G-CSF receptor gene was cloned from the human spleen cDNA library by the RT-PCR technique [Mullis, K., et al. , "Cold Spring Harb Symp. Quant. Biol.", **51**,263-273(1986)]. The human G-CSF receptor gene was inserted into a multi-cloning site EcoRI of an expression vector (pIRESpuro). The expression vector (human G-CSF-R/pIRESpuro) was inserted into *E. coli,* followed by proliferation. The expression vector was then extracted. As host cells, BAF/B03 cell line was used.

The expression vector, human G-CSF-R/pIRESpuro, (20 *µ*g) was added to the host cells BAF/B03 (5 x 10⁷ cells) in K-PBS (800 *µ*L; 30.8 mM NaCl, 120.7 mM KCl, 8.1 mM Na₂HPO₄, 1.46 mM KH₂PO₄, 5 mM MgCl₂), and the resulting mixture was left over at 4°C for 15 minutes. Introduction of the gene was conducted by electroporation at a voltage of 280 V and a capacitance of 950 *µ*F. Subsequent to the introduction of the gene, the cells were suspended in DMEM medium ((+)10% PCS) which contained 10% of a culture (WEHIsup) of a mouse cell line WEHI-3B, said culture containing mouse IL-3, and were incubated. Forty-eight hours later, the cells were suspended in DMEM medium ((+) 10% FCS, 20 ng/mL recombinant human G-CSF (rhG-CSF)) which contained puromycin (2 *µ*g/mL), and were constructed.

Upon determining cytoproliferative activity, a cell line transfected with the human G-CSF receptor gene (BAF/hGCSFR) was recovered and, subsequent to its washing with PBS(-), was suspended in DMEM medium ((-)FCS). The cells were prepared at 5x10⁴ cells/well on a 96-well plate. To the individual wells, rhG-CSF was added at 0, 0.025, 0.1, 0.4, 1.6 or 6.4 ng/mL, respectively, followed by incubation at 5% CO₂ and 37°C for 48 hours. Subsequent to the incubation, a WST-1/1-methoxy PMS solution (product of Dojindo Laboratories) was added to the individual wells (final concentration: 5 mM), and a staining reaction was then conducted in a CO₂ incubator. After the reaction, measurement was conducted at a measurement wavelength of 450 nm.

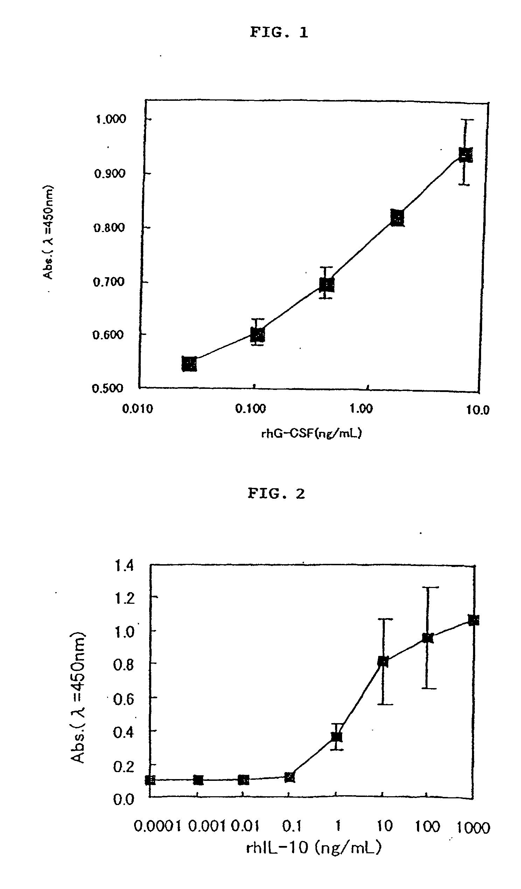

As a result, the cell line (BAF/hGCSFR) transfected with the human G-CSF receptor gene was found to give good linearity in an rhG-CSF concentration range of from 0.025 to 6.4 ng/mL (FIG. 1). Therefore, it has become evident that the method of the present invention permits a quantitative assay of hG-CSF or an hG-CSF-like low molecular compound.

### Example 2

### Construction of a human IL-10-dependent cell line

Using a primer prepared based on a human IL-10 receptor gene, a human IL-10 receptor gene was cloned from the human spleen cDNA library by the RT-PCR technique [Mullis, K., et al., "Cold Spring Harb Symp. Quant. Biol.", **51**, 263-273 (1986)]. The human IL-10 receptor gene was inserted into the multi-cloning site Bam HI of the expression vector (pIRESpuro). The expression vector (human IL-10/pIRESpuro) was inserted into *E*. *coli,* followed by proliferation. The expression vector was then extracted.

The expression vector, human IL-10-R/pIRESpuro, (20 *µ*g) was added to the host cells BAF/B03 (5 x 10⁷ cells) in K-PBS (800 *µ*L; 30.8 mM NaCl, 120.7 mM KCl, 8.1 mM Na₂HPO₄, 1.46 mM KH₂PO₄, 5 mM MgCl₂), and the resulting mixture was left over at 4°C for 15 minutes. Introduction of the gene was conducted by electroporation at a voltage of 280 V and a capacitance of 950 *µ*F. Subsequent to the introduction of the gene, the cells were suspended in DMEM medium ((+)10% FCS) which contained 10% of a culture (WEHIsup) of a mouse cell strain WEHI-3B, said culture containing mouse IL-3, and were incubated. Forty-eight hours later, the cells were suspended in DMEM medium ((+) 10% FCS, 20 ng/mL recombinant human IL-10 (rhIL-10)) which contained puromycin (2 *µ*g/mL), and were constructed.

Upon determining cytoproliferative activity, a cell line transfected with the human IL-10 receptor gene (BAF/hIL-10R was recovered and, subsequent to its washing with PBS(-), was suspended in DMEM medium ((-)FCS). The cells were prepared at 5x10⁴ cells/well on a 96-well plate. To the individual wells, rhIL-10 was added at 0, 0.0001, 0.001, 0.01, 0.1, 1, 10, 100 or 1,000 ng/mL, respectively, followed by incubation at 5% CO₂ and 37°C for 48 hours. Subsequent to the incubation, the WST-1/1-methoxy PMS solution (product of Dojindo Laboratories) was added (final concentration: 5 mM), and a staining reaction was then conducted in a CO₂ incubator. After the reaction, measurement was conducted at a measurement wavelength of 450 nm.

As a result, the cell line (BAF/hIL-10R) transfected with the human IL-10 receptor gene was confirmed to undergo cytoproliferation commensurate with its added amount when rhIL-10 is added at 0.1 to 1,000 ng/mL (FIG. 2). Therefore, it has become evident that the method of the present invention permits an assay of hIL-10 or an hIL-10-like low molecular compound.

### Example 3

### Effects of a reducing agent on proliferation of a G-CSF-dependent cell line

With respect to the cell line (BAF/hGCSFR) obtained in Example 1 with the human G-CSF receptor gene introduced therein, effects which may be given to its screening by the existence of a reducing agent (2-mercaptoethanol) was investigated as will be described next. Firstly, two culture media were provided, one being a culture medium obtained by adding 2-mercaptoethanol at 5 x 10⁻⁵ M to DMEM medium ((-)FCS) and the other consisting solely of DMEM medium ((-)FCS) without incorporation of the additives. Those culture media were poured into individual wells of 96-well plates, respectively, and BAF/hG-CSFR which had been washed with PBS(-) was suspended at 5 x 10⁴ cells/well.

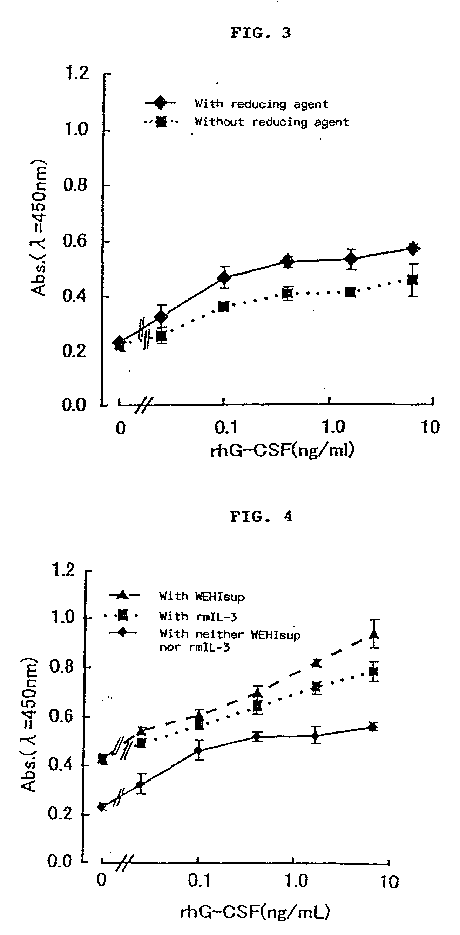

To the individual wells, rhG-CSF was then added at 0, 0.025, 0.1, 0.4, 1.6 or 6.4 ng/mL, respectively, followed by incubation at 5% CO₂ and 37°C for 48 hours. Subsequent to the incubation, the WST-1/1-methoxy PMS solution (product of Dojindo Laboratories) was added to the individual wells (final concentration: 5 mM), and a staining reaction was then conducted in a CO₂ incubator. As a result, cytoproliferation of BAF/hG-CSFR in the presence of rhG-CSF was enhanced by the addition of 2-mepcaptoethanol, a reducing agent, upon incubation of the cell line. Further, marked differences in cytoproliferation were observed at the rhG-CSF concentrations of 0.1, 0.4, 1.6 and 6.4 ng/mL, respectively. The results are shown in FIG. 3.

### Example 4

### Effects of IL-3 on proliferation of a G-CSF-dependent cell line

With respect to the cell line (BAF/hGCSFR) obtained in Example 1 with the human G-CSF receptor gene introduced therein, effects which may be given to its screening by the existence of IL-3 was investigated as will be described next. Firstly, three culture media were provided, including a culture medium obtained by adding the mouse recombinant IL-3 at a final concentration of 0.5 ng/mL to DMEM medium ((-)FCS, added with 2-mercarptoethanol at a final concentration of 5 x 10⁻⁵ M), another culture medium obtained by adding a culture (WEHIsup) of the mouse cell line WEHI-3B, said culture also containing the mouse recombinant IL-3, at a final concentration of 1.25%, and a further culture medium consisting solely of DMEM medium ((-)FCS) without incorporation of those additives.

Those culture media were poured into individual wells of 96-well plates, respectively, and BAF/hG-CSFR which had been washed with PBS(-) was suspended at 5 x 10⁴ cells/well in the respective wells. To the individual wells, rhG-CSF was then added at 0, 0.025, 0.1, 0.4, 1.6 or 6.4 ng/mL, respectively, followed by incubation at 5% CO₂ and 37°C for 48 hours.

Subsequent to the incubation, the WST-1/1-methoxy PMS solution (product of Dojindo Laboratories) was added to the individual wells (final concentration: 5 mM), and a staining reaction was then conducted in a CO₂ incubator. As a result, cytoproliferation of BAF/hG-CSFR in the presence of rhG-CSF was enhanced by the addition of the mouse recombinant IL-3 and WEHIsup upon incubation of the cell line. When a comparison was made in cytoproliferation upon addition of the mouse recombinant IL-3 and WEHIsup, marked differences in cytoproliferation were observed at the rhG-CSF concentrations of 1.6 and 6.4 ng/mL, respectively. The results are shown in FIG. 4.

### Example 5

### Effects of a reducing agent and IL-3 on proliferation of a G-CSF-dependent cell line in which the G-CSF is a G-CSF-like low molecular substance

With respect to the cell line (BAF/hGCSFR) obtained in Example I with the human G-CSF receptor gene introduced therein, effects which may be given to its screening by the existence of IL-3 was investigated as will be described next. Firstly, two culture media were provided, one being a culture medium obtained by adding a culture (WEHIsup) of the mouse cell line WEHI-3B at a final concentration of 1.25% to DMEM medium ((-)FCS; added with 2-mercaptoethanol at a final concentration of 5 x 10⁻⁵ M) and the other consisting solely of DMEM medium ((-)FCS )without incorporation of those additives.

Those culture media were poured into individual wells of 96-well plates, respectively, and BAF/hG-CSFR which had been washed with PBS(-) was suspended at 5 x 10⁴ cells/well in the respective wells. To the individual wells, SB247464 [a low molecular compound reported as a substitute for G-CSF by Smithkline Beecham Seiyaku K.K.; see "Science", **281**(5374): 257-9(1998)] was then added at 0, 10⁻¹⁰, 10⁻⁹, 10⁻⁸, 10⁻⁷ and 10⁻⁶ M, respectively, followed by incubation at 5% CO₂ and 37°C for 48 hours.

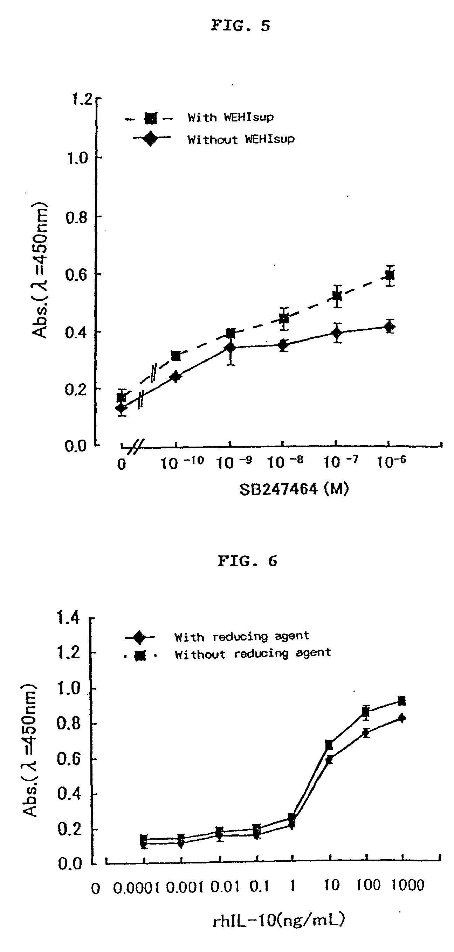

Subsequent to the incubation, the WST-1/1-methoxy PMS solution (product of Dojindo Laboratories) was added to the individual wells (final concentration: 5 mM), and a staining reaction was then conducted in a CO₂ incubator. As a result, cytoproliferation of BAF/hG-CSFR in the presence of SB247464 was enhanced by the addition of WEHI sup to the culture medium. Further, marked differences in cytoproliferation were observed at the SB247464 concentrations of 10⁻⁸, 10⁻⁹ and 10⁻¹⁰_{,} respectively. The results are shown in FIG. 5.

### Example 6

### Effects of a reducing agent on proliferation of the IL-10-dependent cell line

With respect to the cell line (BAF/hIL-10R obtained in Example 2 with the human IL-10 receptor gene introduced therein, effects which may be given to its screening by the existence of a reducing agent (2-mercaptoethanol) was investigated as will be described next. Firstly, two culture media were provided, one being a culture medium obtained by adding 2-mercaptoethanol at 5 x 10⁻⁵ M to DMEM medium ((-)FCS) and the other consisting solely of DMEM medium ((-)FCS) without addition of the reducing agent. Those culture media were poured into individual wells of 96-well plates, respectively, and BAF/hIL-10R which had been washed with PBS(-) was suspended at 5 x 10⁴ cells/well.

To the individual wells, rhIL-10 was then added at 0, 0.0001, 0.001, 0.01, 0.1, 1, 10, 100 and 1,000 ng/mL, respectively, followed by incubation at 5% CO₂ and 37°C for 48 hours. Subsequent to the incubation, the WST-1/1-methoxy PMS solution (product of Dojindo Laboratories) was added to the individual wells (final concentration: 5 mM), and a staining reaction was then conducted in a CO₂ incubator. As a result, cytoproliferation of BAF/hIL-10R in the presence of rhIL-10 was enhanced by the addition of 2-mepcaptoethanol, a reducing agent, upon incubation of the cell line. Further, marked differences in cytoproliferation were observed at the rhIL-10 concentrations of 10, 100 and 1,000 ng/mL, respectively. The results are shown in FIG. 6.

### Example 7

### Effects of IL-3 on proliferation of the IL-10-dependent cell line

With respect to the cell line (BAF/hIL-10R) obtained in Example 2 with the human IL-10 receptor gene introduced therein, effects which may be given to its screening by the existence of IL-3 was investigated as will be described next. Firstly, three culture media were provided, including a culture medium obtained by adding the mouse recombinant IL-3 at a final concentration of 2 ng/mL to DMEM medium ((-)FCS, added with 2-mercarptoethanol at a final concentration of 5 x 10⁻⁵ M), another culture medium obtained by adding a culture (WEHIsup) of the mouse cell line WEHI-3B at a final concentration of 0.625%, and a further culture medium consisting solely of DMEM medium ((-)FCS) without incorporation of those additives.

Those culture media were poured into individual wells of 96-well plates, respectively, and BAF/hIL-10R which had been washed with PBS(-) was suspended at 5 x 10⁴ cells/well in the respective wells. To the individual wells, rhIL-10 was then added at 0, 0.025, 0.1, 0.4, 1.6 or 6.4 ng/mL, respectively, followed by incubation at 5% CO₂ and 37°C for 48 hours.

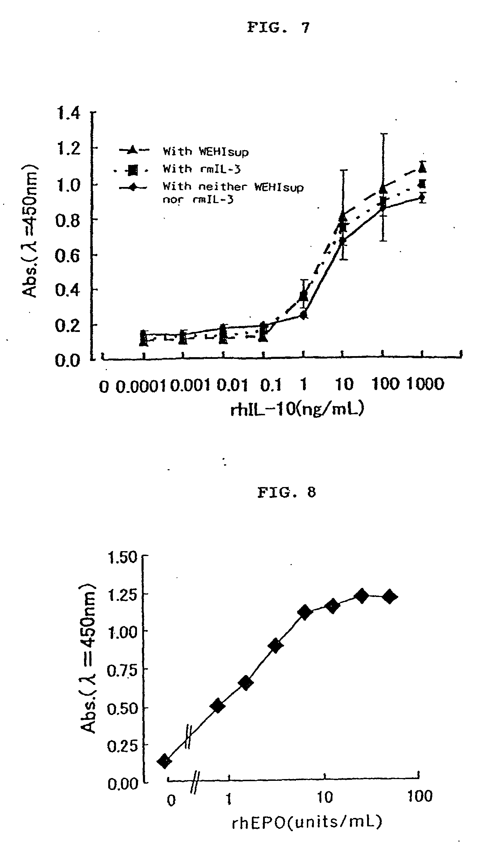

Subsequent to the incubation, the WST-1/1-methoxy PMS solution (product of Dojindo Laboratories) was added to the individual wells (final concentration: 5 mM), and a staining reaction was then conducted in a CO₂ incubator. As a result, cytoproliferation of BAF/hIL-10R in the presence of rIL-10 was enhanced by the addition of the mouse recombinant IL-3 and WEHIsup upon incubation of the cell line. The results are shown in FIG. 7.

### Example 8

### Construction of a human EPO-dependent cell line

A cDNA library was prepared from a human erythroid leukemia cell line. Using a primer prepared based on a human EPO receptor gene, a human EPO receptor gene was cloned from the cDNA library by the RT-PCR technique [Mullis, K., et al., "Cold Spring Harb Symp. Quant. Biol.", **51**, 263-273 (1986)]. The human EPO receptor gene was inserted into the multi-cloning site EcoRI of the expression vector (pIRESpuro). The expression vector (human EPO-R/pIRESpuro) was inserted into *E. coil,* followed by proliferation. The expression vector was then extracted. As host cells, BAF/B03 cell line was used [Palacios, R., et al., "Cell", **41**, 727-734 (1985); Shi, Y., et al., "Immunol.", **159**, 5318-5328 (1997)].

The expression vector, human EPO-R/pIRESpuro, (20 *µ*g) was added to the host cell line BAF/B03 (5 x 10⁷ cells) in K-PBS (800 *µ*L; 30.8 mM NaCl, 120.7 mM KCl, 8.1 mM Na₂HPO₄, 1.46 mM KH₂PO₄, 5 mM MgCl₂), and the resulting mixture was left over at 4°C for 15 minutes. Introduction of the gene was conducted by electroporation at a voltage of 280 V and a capacitance of 950 *µ*F. Subsequent to the introduction of the gene, the cells were suspended in DMEM medium ((+) 10% FCS) which contained mouse IL-3, and were incubated. Forty-eight hours later, the cells were suspended in DMEM medium ((+) 10% FCS, 250 units/mL recombinant human EPO (rhEPO)) which contained puromycin (2 *µ*g/mL,), and a cell line (BAF/hEPOR) was constructed.

Upon determining cytoproliferative activity, a cell line transfected with the human EPO receptor gene (BAF/hEPOR) was recovered and, subsequent to its washing with PBS(-), was suspended in DMEM medium ((-)FCS). The cells were prepared at 5x10⁴ cells/well on a 96-well plate. To the individual wells, rhEPO was added at 0, 0.8, 1.6, 3.1, 6.3, 12.5, 25 or 50 units/mL, respectively, followed by incubation at 5% CO₂ and 37°C for 48 hours. Subsequent to the incubation, a WST-1/1-methoxy PMS solution (product of Dojindo Laboratories) was added to the individual wells (final concentration: 5 mM), and a staining reaction was then conducted in a CO₂ incubator. After the reaction, measurement was conducted at a measurement wavelength of 450 nm.

As a result, the cell line (BAF/EPOR) transfected with the human EPO receptor gene was found to give good linearity in an rhEPO concentration range of from 0.8 to 50 units/mL (FIG. 8). Therefore, it has become evident that the method of the present invention permits a quantitative assay of hEPO or an hEPO-like low molecular compound.

### Example 9

### Effects of a reducing agent on proliferation of the EPO-dependent cell line

With respect to the cell line (BAF/hEPOR) obtained in Example 8 with the human EPO receptor gene introduced therein, effects which may be given to its screening by the existence of a reducing agent (2-mercaptoethanol) was investigated as will be described next. Firstly, two culture media were provided, one being a culture medium obtained by adding 2-mercaptoethanol at 5 x 10⁻⁵ M to DMEM medium ((-)FCS) and the other consisting solely of DMEM medium ((-)FCS) without addition of the reducing agent. Those culture media were poured into individual wells of 96-well plates, respectively, and BAF/hEPOR which had been washed with PBS(-) was suspended at 5 x 10⁴ cells/well.

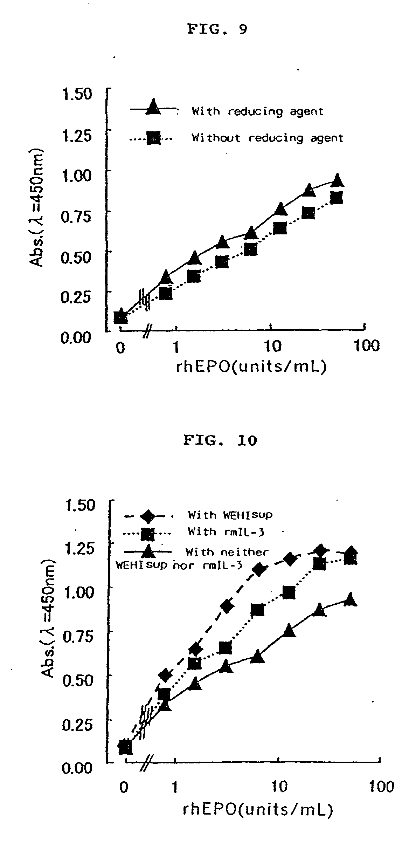

To the individual wells, rhEPO was then added at 0, 0.8, 1.6, 3.1, 6.3, 12.5, 25 or 50 units/mL, respectively, followed by incubation at 5% CO₂ and 37°C for 48 hours. Subsequent to the incubation, the WST-1/1-methoxy PMS solution (product of Dojindo Laboratories) was added to the individual wells (final concentration: 5 mM), and a staining reaction was then conducted in a CO₂ incubator. After the reaction, measurement was conducted at a measurement wavelength of 450 nm. As a result, cytoproliferation of BAF/hEPOR in the presence of rhEPO was enhanced by the addition of 2-mepcaptoethanol, a reducing agent, upon incubation of the cell line. The results are shown in FIG. 9.

### Example 10

### Effects of IL-3 on proliferation of the EPO-dependent cell line

With respect to the cell line (BAF/hEPOR) obtained in Example 8 with the human EPO receptor gene introduced therein, effects which may be given to its screening by the existence of IL-3 was investigated as will be described next. Firstly, three culture media were provided, including a culture medium obtained by adding the mouse recombinant IL-3 at a final concentration of 0.5 ng/mL to DMEM medium ((-)FCS, added with 2-mercarptoethanol at a final concentration of 5 x 10⁻⁵ M), another culture medium obtained by adding a culture (WEHIsup) of the mouse cell line WEHI-3B, said culture also containing the mouse recombinant IL-3, at a final concentration of 1.25%, and a further culture medium consisting solely of DMEM medium ((-)FCS) without incorporation of those additives.

Those culture media were poured into individual wells of 96-well plates, respectively, and BAF/hEPOR which had been washed with PBS(-) was suspended at 5 x 10⁴ cells/well in the respective wells. To the individual wells, rhEPO was then added at 0, 0.8, 1.6, 3.1, 6.3, 12.5, 25 or 50 units/mL, respectively, followed by incubation at 5% CO₂ and 37°C for 48 hours.

Subsequent to the incubation, the WST-1/1-methoxy PMS solution (product of Dojindo Laboratories) was added to the individual wells (final concentration: 5 mM), and a staining reaction was then conducted in a CO₂ incubator. After the reaction, measurement was conducted at a measurement wavelength of 450 nm. As a result, cytoproliferation of BAF/hEPOR in the presence of rhEPO was enhanced by the addition of the mouse recombinant IL-3 and WEHIsup upon incubation of the cell line. When a comparison was made in cytoproliferation upon addition of the mouse recombinant IL-3 and WEHIsup, marked differences in cytoproliferation were observed at the rhEPO concentrations of 3.1, 6.3 and 12.5 units/mL, respectively. The results are shown in FIG. 10.

Other ligand receptor genes can also be formed into genes, which are to be inserted, by cloning them by the RT-PCR technique and inserting the cloned genes into expression vectors as in Example 1, 2 or 8. Following the procedures of Example 1, 2 or 8, the genes so formed can also be introduced into target cells by applying electroporation or the like, and cell lines capable of undergoing proliferation against the corresponding ligands or ligand-like low molecular substances can be constructed. Further, in accordance with the description of Examples 3 to 7 or Example 9 or 10, the ligands or ligand-like low molecular compounds can be detected using the cell lines, respectively.

## Claims

1. A method for assaying a ligand or a ligand-like low molecular compound in a test compound, which comprises separating a ligand-dependent cell line, which has been transfected with a ligand receptor gene and an antibiotic-resistant gene, from other cell lines, culturing said ligand-dependent cell line in the presence of said test compound, and then determining cytoproliferative activity of said ligand-dependent cell line.

2. A method according to claim 1, wherein said culture of said ligand-dependent cell line transfected with said ligand receptor gene and said antibiotic-resistant gene is conducted in the presence of said test compound and a reducing agent.

3. A method according to claim 2, wherein said reducing agent is 2-mercaptoethanol or α-thioglycerol.

4. A method according to claim 1, wherein said culture of said ligand-dependent cell line transfected with said ligand receptor gene and said antibiotic-resistant gene is conducted in the presence of said test compound, a reducing agent and interleukin 3.

5. A method according to any one of claims 1-4, wherein said ligand receptor gene is human granulocyte colony stimulating factor receptor gene, and said ligand or said ligand-like low molecular compound in said test compound is human granulocyte colony stimulating factor or a low molecular compound usable in place of human granulocyte colony stimulating factor.

6. A method according to any one of claims 1-4, wherein said ligand receptor gene is human interleukin 10 receptor gene, and said ligand or said ligand-like low molecular compound in said test compound is human interleukin 10 or a low molecular compound usable in place of human interleukin 10.

7. A method according to any one of claims 1-4, wherein said ligand receptor gene is human erythropoietin receptor gene, and said ligand or said ligand-like low molecular compound in said test compound is human erythropoietin or a low molecular compound usable in place of human erythropoietin.

8. A method according to any one of claims 1-4, wherein said ligand receptor gene is human thrombopoietin receptor gene, and said ligand or said ligand-like low molecular compound in said test compound is human thrombopoietin or a low molecular compound usable in place of human thrombopoietin.

9. A method according to any one of claims 1-4, wherein said ligand receptor gene is human insulin receptor gene, and said ligand or said ligand-like low molecular compound in said test compound is human insulin or a low molecular compound usable in place of human insulin.

10. A method according to any one of claims 1-4, wherein said ligand receptor gene is human nerve growth factor receptor gene, and said ligand or said ligand-like low molecular compound in said test compound is human nerve growth factor or a low molecular compound usable in place of human nerve growth factor.

11. A method according to any one of claims 1-4, wherein said antibiotic-resistant gene is a gene having resistance to an antibiotic having protein synthesis inhibiting effect.

12. A method according to claim 1 or 8, wherein said antibiotic-resistant gene is a puromycin-resistant gene, a hygromycin-resistant gene or a neomycin-resistant gene.

13. A method according to any one of claims 1-4, wherein said cytoproliferative activity of said ligand-dependent cell line is determined by causing a colorimetric substance and an electron carrier on said ligand-dependent cell line.

14. A method according to claim 13, wherein said colorimetric substance is a tetrazolium salt, and said electron carrier is a phenazine methosulfate electron carrier.

15. A method according to claim 14, wherein said colorimetric substance is WST-1, and said electron carrier is 1-methoxy PMS.

16. An assay reagent for a ligand or a ligand-like low molecular compound, which comprises the following components (a)-(d):
(a) a ligand-dependent cell line transfected with a ligand receptor gene and an antibiotic-resistant gene;
(b) a culture medium for said ligand-dependent cell line;
(c) a colorimetric substance; and
(d) an electron carrier.

17. An assay reagent according to claim 16, wherein said component (b) comprises a reducing agent and/or interleukin 3.

18. An assay kit for a ligand or a ligand-like low molecular compound, which comprises one or more of the following components (a)-(d):
(a) a ligand-dependent cell line transfected with a ligand receptor gene and an antibiotic-resistant gene;
(b) a culture medium for said ligand-dependent cell line;
(c) a colorimetric substance; and
(d) an electron carrier.

19. An assay kit according to claim 18, wherein said component (b) comprises a reducing agent and/or interleukin 3.
